(19) Europäisches Patentamt
European Patent Office
Office européen des brevets

(11) EP 1 834 955 A1

(12) EUROPEAN PATENT APPLICATION

(43) Date of publication:
**19.09.2007 Bulletin 2007/38**

(21) Application number: **06075595.6**

(22) Date of filing: **10.03.2006**

(51) Int Cl.:
***C07D 487/22*** *(2006.01)* ***A61K 31/40*** *(2006.01)*
***A61P 35/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicants:
- **Humboldt Universität zu Berlin
10099 Berlin (DE)**
- **Friedrich-Alexander-Universität Erlangen-Nürnberg
91054 Erlangen (DE)**

(72) Inventors:
- **Röder, Beate
14612 Falkensee (DE)**
- **Ermilov, Eugeny
12557 Berlin (DE)**
- **Jux, Norbert
91094 Langensendelbach (DE)**
- **Jasinski, Stefan
91338 Igensdorf (DE)**

(74) Representative: **Ziebig, Marlene et al
Gulde Hengelhaupt Ziebig & Schneider
Patentanwälte Rechtsanwälte
Wallstrasse 58/59
10179 Berlin (DE)**

(54) **Porphyrin derivates and their use as photosensitizers in photodynamic therapy**

(57) The present invention relates to novel porphyrin derivates of formula I, especially to porphyrins which carry substituted phenyl moieties in mesoposition. The porphyrin derivates of the invention are useful as photosensitizers in photodynamic therapy (PDT), especially in photodynamic tumor therapy. Due to their excellent photophysical properties the compounds of formula I are extremely efficient in photodynamic treatment.

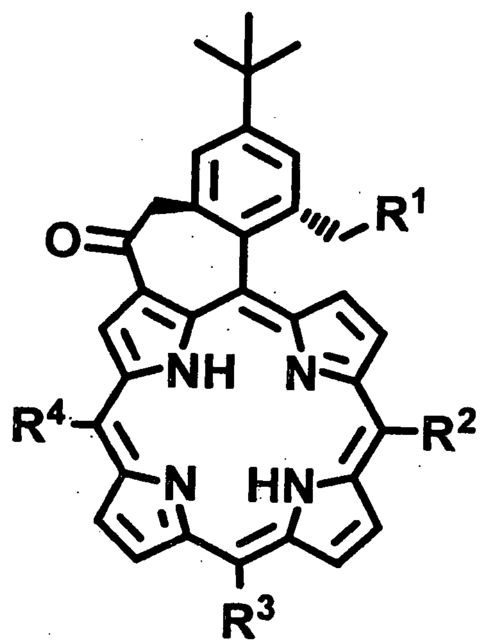

(I)

EP 1 834 955 A1

## Description

The present invention relates to novel porphyrin derivates of formula I, especially to porphyrins with four substituted phenyl moieties in meso-position. The porphyrin derivatives of the invention are useful as photosensitizers in photodynamic therapy (PDT), especially in photodynamic tumor therapy. Due to their excellent photophysical properties the compounds of formula I are extremely efficient in photodynamic treatment.

Photodynamic treatment is a procedure that uses photoselective (light-activated) compounds to target and destroy diseased cells. The photoselective compounds which are also called photosensitizers are inactive until switched on by light of a specific wavelength. By absorbing the light, energy is transferred from the photosensitizer to molecular oxygen generating singlet molecular oxygen ($^1O_2$) which plays the key role in PDT and causes direct or indirect photodamage of the target tissue.

A large number of naturally occurring and synthetic dyes have been evaluated as potential photosensitizers in photodynamic therapy. Perhaps the most widely studied class of photosensitizers are the tetrapyrrolic macrocyclic compounds. A photosensitizer developed on porphyrin basis is Photofrin® (Axcan Pharma Inc., US) currently being used in clinical treatment. Photofrin® is an oligomer mixture of hematoporphyrins having different pharmacokinetic properties. Additionally, Photofrin® processes a non-comfortable absorption maximum which is in the range of the absorption of haemoglobin.

WO 01/74818 describes chlorines as photoselective compounds for photodynamic therapy. Chlorins are compounds that differ from porphyrins in that one of the pyrrole rings has been reduced, either by addition of hydrogen or by carbon bond formation. Chlorins have an increased absorption in the red region which is very attractive for PDT. But the general disadvantage of these compounds is their sensitivity to oxidation under physiological conditions. This also applies to m-tetrahydroxyphenylchlorin whose in vivo activity has been studied intensely (Blant, S. A. et. al., "In vivo fluence rate effect in photodynamic therapy of early cancers with tetra(m-hydroxyphenyl)chlorin", Photochem. Photobiol. 64, 963-968 (1996)).

A further group of known photosensitizers are the phorbides. These compounds are tetrapyrroles derived from the naturally occurring chlorophyll and having absorptions around 670 nm. A main representative of this group is pheophorbide a whose photodynamic activity has been shown in vitro and in vivo (compare e. g. DD 248 282 A1). Phorbides have the disadvantage that their synthesis is quite difficult and requires vast resources.

Further potential photosensitizers derived from meso-tetraarylporphyrins are described from Richeter, S. et. al. in J. Org. Chem. 2003, 68, 9200-9208. The described compounds are oxonaphthoporphyrins, where one pyrrole and a neighboring aryl group are fused with a keto function, or dioxoanthroporphyrins, where two pyrrols and the aryl group in meso-position between them are fused each with a keto function. The diketones show a bathochromic shift of the UVvis absorption, the longest wave-length bands absorbing in the 700-825 nm range.

It is an object of the present invention to provide photosensitizers for PDT treatment having excellent light absorption properties and, especially, being able to generate high singlet oxygen quantum yields under irradiation.

It is another object of the present invention to provide photosensitizers which are stable under physiological conditions, soluble in water and completely obtainable by chemical synthesis.

It is still another object of the present invention to provide an efficient method of photodynamic therapy, especially for cancer treatment.

The purpose of the present invention is achieved by providing the novel porphyrin derivates of formula I

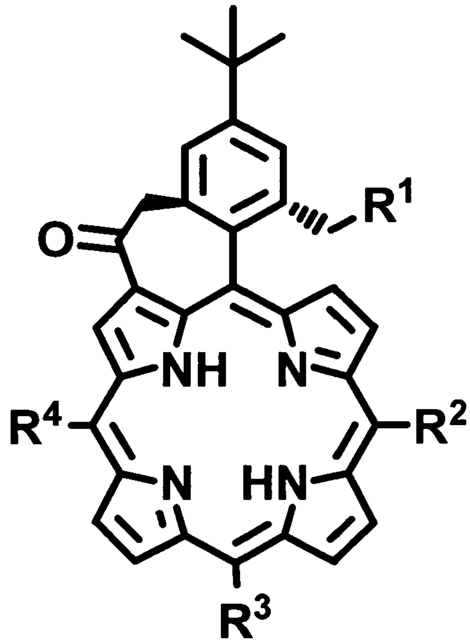

(I)

wherein

$R^1$ represents hydrogen, $COR^5$, $CO_2R^5$, $CONR^5R^6$ $SO_2R^5$, $SO_2NR^5R^6$ or

$R^1$ represents a carboxy group whose C-atom is covalently bound to the C-atom of position 7 of the porphyrin chromophor forming a keto function;

$R^2$, $R^3$ and $R^4$ are independently selected from unsubstituted or substituted $C_1$-$C_6$ alkyl, unsubstituted or substituted aryl;

$R^5$ and $R^6$ are independently selected from hydrogen, unsubstituted or substituted $C_1$-$C_6$ alkyl, unsubstituted or substituted aryl, halogen, $C_1$-$C_5$ alkoxy, alkylether, carboxylic acid or acid salts, carboxylic acid esters, amino, nitro, amino acids or their derivates, a dendrimer moiety, a micelle forming detergent, a lipid, an antibody or antibody fragment, polypropylene glycol, polyethylene glycol (PEG), 2-hydroxypropyl methacrylate, polyvinylpyrrolidone (PVP), a pharmaceutically acceptable salt.

The inventors of the present invention discovered that the compounds of formula I show excellent photophysical properties. Their absorption maximum is in the range of 680-700nm, that means far from the absorption maximum of haemoglobin (613 nm). But above all, the compounds of formula I possess a high triplet quantum yield of about 95 % and long triplet lifetime so that superior singlet molecular oxygen quantum yields of about 90 % are provided. As it can be taken from Encyclopedia of Analytical Chemistry, R. A. Meyers (Ed.), pp. 302-322, "Photodynamic Therapy", Table 2, John Wiley & Sons Ltd., Chichester, 2000, the singlet molecular quantum yields of porphyrins described there are only about 50 to 60 %.

That means, the compounds of formula I have been found as being excellent photosensitizers for photodynamic therapy because they possess the following properties:

a) they are stable under physiological conditions,
b) they are well-soluble compounds, by adapting $R^1$ - $R^4$ the water solubility can be adjusted,
c) they are obtainable by simple chemical synthesis,
d) under irradiation they generate singlet oxygen quantum yields of about 90% ensuring a cell killing effect against unwanted tissues, especially tumors.

In a preferred embodiment the present invention relates to compounds according to formula I, wherein $R^1$ is hydrogen or wherein $R^1$ represents a carboxy group which C-atom is covalently bound to the C-atom of position 7 of the porphyrin macrocycle.

It is further preferred that $R^2$, $R^3$ and $R^4$ are independently selected from an unsubstituted or substituted aryl group, especially phenyl or phenyl which is substituted with tert-butyl. In a more preferred embodiment $R^2$, $R^3$ and $R^4$ are 4-tert-butylphenyl.

In another embodiment of the invention $R^2$, $R^3$ and $R^4$ independently from each other represent $C_1$-$C_6$ alkyl, preferably $C_1$-$C_4$ alkyl.

In a most preferred embodiment the present invention relates to compounds FB-1-CO ($R^1$=hydrogen, $R^2$=$R^3$=$R^4$=4-t-butylphenyl) and FB-2-CO (R1 is a carboxy group whose C-atom is linked with the C-atom of position 7 of the neighboring pyrrole of the porphyrin macrocycle). These special compounds are depicted in Fig. 4.

The tetraaryl compounds of formula I have been prepared starting with the known Zn-complexes of tetraaryl porphyrin systems bearing in $X^2$- and/or $X^6$-position (X=5,15) bromomethyl groups ["o-(Bromomethyl)Substituted Tetraarylporphyrin Building Blocks" N. Jux, Org. Lett. 2000, 2, 2129-2132; "Introducing the Staudinger phosphazene reaction to porphyrin chemistry" Nga Hoang Huyen, Ulrike Jannsen, Hanaa Mansour, Norbert Jux, J. Porphyrins Phthalocyanines 2004). The syntheses have been carried out with derivates having up to four bromomethyl groups. The synthesis of mono, di, tri and tetraphenyl porphyrins is well known in the art (compare "The Porphyrins" Ed. D. Dolphin, Academic Press, 1978, Volume 1, chapter 3, 88-90; Gunter, M. J., Mander, L. N., J. Org. Chem. 46, 4792-4795, 1981). Such compounds can be widely functionalized as the aromatic rings may possess different substituents.

Below, as non-limiting examples for the synthesis of the compounds of formula I the preparation of FB-1-CO and FB-2-CO is described in detail. Other compounds of formula I are prepared in analogy starting with compounds having the desired substituents in the porphyrin macrocyclus. The synthesis of porphyrins with $R^2$, $R^3$ and $R^3$ = alkyl is performed in a similar fashion with aliphatic aldehydes as starting materials. Typical procedures are outlined in the literature (an excellent summary can be found in K. M. Kadish, K. M. Smith, R. Guilard (Eds.): J. S. Lindsey, The Porphyrin Handbook, Vol. 1, Chapter 2: "Synthesis of meso-Substituted Porphyrins", Academic Press, San Diego 2000, pp 45-112.

In still another preferred embodiment of the invention $R^1$ in formula I represents a carbonyl, carboxyl or amino group which can be covalently bound to a biologically active group, for instance dendrimer(s), lipid(s), micelle forming agent(s), antibodies or antibody fragments to promote the transport and/or the selective accumulation of the photosensitizers of formula I into target tissue. Suitable dendrimers are for instance described in S. Hackbarth et. al., Optics Communications 248 (2005), 295-306 and WO 01/08704 A2. To these dendrimers a large number of compounds of formula I can be attached forming a carrier unit to deliver the photosensitizers to the target tissue. As micelle forming agents all known tensides are applicable, for instance long chain fatty acids. Preferred lipids for linking are glycolipids and phospholipids, especially glycerophospholipids and LDL. Antibodies or antibody fragments which can be covalently bound to $R^1$ are preferably monoclonal antibodies and they are selected to have sufficient affinity to the antigen of the tissue to be treated. Amino acids or their derivates are also useful biologically active substituents, such as those derived for instance from valine, leucine, isoleucine, threonine, methionine, phenylalanine, tryptophan, alanine, aspartic acid, cystine, cysteine, glutamic acid, glycine, histidine, proline, serine, tyrosine, asparagine and glutamine.

According to the invention pharmaceutically acceptable or physiologically tolerable salts of compounds of formula I are alkali or alkaline earth salts, particularly sodium, potassium, calcium or ammonium salts.

In the meaning of the invention, alkyl represents a branched or unbranched hydrocarbon chain. Thus, $C_1$-$C_4$ alkyl for instance includes methyl, ethyl, n-propyl, i-propyl, n-butyl, i-butyl, sec-butyl and tert-butyl. "Aryl" represents in the meaning of the invention substituted or unsubstituted phenyl, naphthyl or anthryl rings.

According to the invention "substituted alkyl or aryl" means substituted with alkoxy, hydroxy, halogen, amino or carboxy groups. Alkoxy represents a branched or unbranched hydrocarbon chain with one or more intervening oxygen atoms.

The present invention also relates to the compounds of formula I for use as photosensitizers in photodynamic therapy of unwanted tissue, tumor, cancer and malignant tissue. The compounds of formula I can be used for the therapeutic treatment of a broad range of tumors. Examples of such tumors are breast cancer, skin cancer, especially basal cell carcinoma, lung cancer, cancer of the urinary bladder, esophageal cancer, gastric cancer, enteric cancer, uterine cancer, ovarian cancer, pancreatic cancer, sarcomas, hepatic cancer, cancer of the upper jaw, cancer of the bile duct, cerebral tumor, malignant goiter, prostatic cancer, cancer of the parotid gland, Hodgkin's disease, multiple myeloma, renal cancer, leukaemia, and malignant lymphocytoma.

The compounds of the present invention are also useful for the treatment of ophtalmological disorders such as age-related macula degeneration, diabetic retinopathy and choroidal neovascularization; dermatological disorders such as psoriasis; urological disorders such as condyloma virus and cardiovascular disorders such as restenosis and atherosclerotic plaques. The compounds of formula I can also be used for the treatment of viral infections, especially Herpes simplex, or bacterial infections, especially infections with E. coli, Candida, Streptococcus and generally gram positive bacteria.

Therefore, the present invention also relates to a method for treating a target tissue in a mammal comprising administering to a mammal an effective amount of at least one compound of formula I which is selectively taken up and/or retained by the tissue and delivering after a certain period of time light of the appropriate wavelength to the target tissue to be absorbed by the photoselective compounds of formula I. This activating light excites the photosensitizer of formula I to generate singlet molecular oxygen destroying the tissue. The source of irradiation for this phototherapy is not limited, but a laser beam is preferable because intensive light rays in the described wavelength range can be

selectively applied. Preferably the irradiation is carried out by laser light from the tip of quartz fibers. Besides the irradiation of the surface of a tumor, the internal part of the tumor can be irradiated by inserting the tip of quartz fibers into the tumor. The irradiation can be visually observed or imaged on a CRT screen.

In the photodynamic treatment the special compounds of formula I as described above are preferably used.

The present invention further concerns the use of compounds of formula I for the preparation of a pharmaceutical composition for photodynamic therapy and the pharmaceutical composition comprising as active ingredient a compound according to formula I. The pharmaceutical composition is adapted to the chosen route of administration, i. e., intravenously, orally, subcutaneously or topically. For this the pharmaceutical composition comprises besides the compound of formula I a pharmaceutically acceptable carrier. As used herein, "a pharmaceutically acceptable carrier" includes any and all solvents, dispersion media, excipients to formulate tablets, pills, capsules, creams, salve formulations, spray solutions or suspensions. In a preferred embodiment of the invention the pharmaceutical compositions for topical administration, e. g. directly to tumors, comprise liposomes, micelles, ointments, gels, hydrogels, and oils. It is well known in the art wow to formulate these pharmaceutical compositions.

The amount of active compound of formula I in such therapeutically useful compositions is such that a suitable dosage will be obtained. In a preferred embodiment the compounds of formula I are administered in a dosage of 0,1 to 5 mg per kilogram body weight/day.

Brief description of the figures

Figure 1 presents the absorption spectrum of FB-1-CO in ethanol (FB-1-CO represents the compound of formula I with $R^1$=hydrogen and $R^2$ = $R^3$ = $R^4$ = 4-t-butylphenyl). The band at 690 nm represents the first exited singlet state of FB-1-CO.

Figure 2 presents the fluorescence spectrum of FB-1-CO. The fluorescence quantum yield has been determined to 0,02.

Figure 3 presents the preferred compounds of formula I of the present invention.

Figure 4 outlines the chemistry involved in the synthesis of FB-1-CO.

Figure 5 outlines the chemistry involved in the synthesis of FB-2-CO.

The following examples are given to highlight a preferred mode of synthesizing compounds of formula I, determining their photophysical properties and their phototoxity in vitro.

## Examples

The preparation of this class of compounds is based on the o-(bromomethyl)-substituted tetraarylporphyrin building blocks developed by Jux ["o-(Bromomethyl)Substituted Tetraaryl-porphyrin Building Blocks" Norbert Jux, Org. Lett. 2000, 2, 2129-2132; "Introducing the Staudinger phosphazene reaction to porphyrin chemistry" Nga Hoang Huyen, Ulrike Jannsen, Hanaa Mansour, Norbert Jux, J. Porphyrins Phthalocyanines 2004, 8, 1356-1365]. The following reaction pathway is illustrated for the preparation of compounds FB-1-CO and FB-2-CO, exemplarily. Other compounds of this class have been synthesized using the same protocols with minor changes concerning amounts of solvents or reaction times. The chemical transformations are based on well known standard methods.

Column chromatography was performed on silica gel 32-63μm, 60 A, MP Biomedicals. $^{1}H$ and $^{13}C$ NMR spectra were recorded on JEOL JMM EX 400, and JEOL GX 400 instruments. FAB mass spectrometry was performed with Micromass Zabspec and Varian MAT 311A machines. Standard UV/VIS spectra were recorded on a Shimadzu UV-3102 PC UV/Vis NIR scanning spectrophotometer. IR spectra were either taken with a Bruker Vector 22 spectrometer or an ASI React IR-1000 spectrometer. Elemental analysis were done with EA 1110 CHNS from CE Instruments. Analytical thin layer chromatography (TLC) was performed on Merck 60 F254 silica gel (precoated on aluminium) with a layer thickness of 0.2 mm.

Example 1

Preparation of $5^4$, $10^4$, $15^4$, $20^4$_tetra-t-butyl [3,$5^2$]-ethano-$5^6$-methyl-$3^1$-oxo-5,10,15,20-tetraphenylporphyrin (FB-1-CO).

*Brief summary of the synthesis of FB-1-CO:*

The mono-bromomethyl porphyrin Zn-1-Br (zinc $5^2$-bromomethyl-$5^4$, $10^4$, $15^4$, $20^4$-tetra-t-butyl-$5^6$-methyl-5,10,15,20-tetraphenylporphyrin) was reacted with potassium cyanide in PEG400 to give the monocyano porphyrin Zn-1-CN (zinc $5^4$, $10^4$, $15^4$, $20^4$-tetra-t-butyl-$5^2$-(cyanomethyl)-$5^6$-methyl-5,10,15,20-tetraphenylporphyrin). Demetallation (FB-1-CN) and acidic saponification of the nitrile substituent gave the monocarboxy porphyrin FB-1-COOH ($5^4$, $10^4$,

$15^4$, $20^4$-tetra-t-butyl-$5^6$-methyl-5,10,15,20-tetraphenylporphyrin-$5^2$-ethanoic acid) which after metallation with cupper (II) chloride gave the copper porphyrin Cu-1-COOH (copper(II) $5^4$, $10^4$, $15^4$, $20^4$-tetra-t-butyl-$5^6$-methyl-5,10,15,20-tetraphenylporphyrin-$5^2$-ethanoic acid). The acid was transformed into the acid chloride (not isolated) with oxalyl chloride and reacted with tin(IV) chloride to give the copper complex Cu-1-CO (copper(II) $5^4$, $10^4$, $15^4$, $20^4$-tetra-t-butyl [3,$5^2$]-ethano-$5^6$-methyl-$3^1$-oxo-5,10,15,20-tetraphenylporphyrin). Demetallation with acid gave the desired free-base system FB-1-CO ($5^4$, $10^4$, $15^4$, $20^4$-tetra-t-butyl [3,$5^2$]-ethano-$5^6$-methyl-$3^1$-oxo-5,10,15,20-tetraphenylporphyrin.

*Protocol:*

**1.** 200 mg (211 μmol) of FB-1-Br ($5^2$-bromomethyl-$5^4$, $10^4$, $15^4$ $20^4$-tetra-t-butyl-$5^6$-methyl-5,10,15,20-tetraphenylporphyrin) were dissolved in 30 ml of dichloromethane. To this solution a saturated solution of 0.93 g (4.22 mmol) zinc acetate ($Zn(O_2CCH_3)_2 \times 2\,H_2O$) in methanol was added and the reaction mixture was stirred at room temperature. When TLC control indicated completion, water was added and the layers were separated. After washing the organic layer three times with water, the residing solution was dried over magnesium sulfate and evaporated to dryness to give Zn-1-Br (zinc $5^2$-bromomethyl-$5^4$, $10^4$, $15^4$, $20^4$-tetra-t-butyl-$5^6$-methyl-5,10,15,20-tetraphenylporphyrin).

**2.** To Zn-1-Br (zinc $5^2$-bromomethyl-$5^4$, $10^4$, $15^4$, $20^4$-tetra-t-butyl-$5^6$-methyl-5,10,15,20-tetraphenylporphyrin), which was obtained by step 1, 546 mg (8.93 mmol) of potassium cyanide and 25 ml polyethylene glycol 400 (PEG 400) were added and the reaction mixture was stirred at room temperature for 24 hours. Afterwards water and dichloromethane were added, the layers separated and the organic layer was finally washed several times with water in order to guarantee absence of free cyanide ions. After evaporation of the solvent, Zn-1-CN (zinc $5^4$, $10^4$, $15^4$, $20^4$-tetra-t-butyl-$5^2$-(cyanomethyl)-$5^6$-methyl-5,10,15,20-tetrapheny-lporphyrin) was obtained as purple crystals.

**3.** To Zn-1-CN (zinc $5^4$, $10^4$, $15^4$, $20^4$-tetra-t-butyl-$5^2$-(cyanomethyl)-$5^6$-methyl-5,10,15,20-tetrapheny-lporphyrin), which was obtained by step 2, half-concentrated aqueous hydrochloric acid was added and the biphasic mixture was vigorously shaken for five minutes. The organic layer was then separated and washed with water, aqueous sodium bicarbonate solution and water. After drying over magnesium sulfate the solution was evaporated to dryness to yield FB-1-CN as a deep violet solid. (Yield: 178 mg, 199 μmol, 94% after steps 1-3).

**4.** FB-1-CN ($5^4$, $10^4$, $15^4$, $20^4$-tetra-t-butyl-$5^2$-(cyanomethyl)-$5^6$-methyl-5,10,15,20-tetrapheny-lporphyrin), which was obtained by step 3, was dissolved in 15 ml of glacial acetic acid. Then 15 ml concentrated sulfuric acid and 5 ml of water were added and the reaction mixture was stirred at 90 °C for 80 hours. Afterwards the mixture was poured on crushed ice and left standing until FB-1-COOH ($5^4$, $10^4$, $15^4$, $20^4$-tetra-t-butyl-$5^6$-methyl-5,10,15,20-tetraphenylporphyrin-$5^2$-ethanoic acid) precipitated completely. The precipitate was filtered of and taken up in dichloromethane. This solution was washed several times with water, dried over magnesium sulfate and finally concentrated to a volume of about 50 ml.

**5.** To this solution of FB-1-COOH ($5^4$, $10^4$, $15^4$, $20^4$-tetra-t-butyl-$5^6$-methyl-5,10,15,20-tetraphenylporphyrin-$5^2$-ethanoic acid), which was obtained by step 4, a solution of 687 mg (3.78 mmol) copper acetate ($Cu(O_2CCH_3)_2$) in 30 ml of methanol and some drops of glacial acetic acid were added and the reaction mixture was stirred for 15 hours at room temperature. Afterwards the reaction mixture was evaporated to dryness and the residue was taken up in dimethyl formamide. The solution was then poured onto a mixture of crushed ice and glacial acetic acid whereupon the copper complex precipitated. After filtration the residue was washed with water, taken up in dichloromethane, the solution dried over magnesium sulfate and finally evaporated to dryness yielding Cu-1-COOH (copper(II) $5^4$, $10^4$, $15^4$, $20^4$-tetra-t-butyl-$5^6$-methyl-5,10,15,20-tetraphenylporphyrin-$5^2$-ethanoic acid) as a bright red powder.

**6.** Cu-1-COOH (copper(II) $5^4$, $10^4$, $15^4$, $20^4$-tetra-t-butyl-$5^6$-methyl-5,10,15,20-tetraphenylporphyrin-$5^2$-ethanoic acid), which was obtained by step 5, was taken up in 50 ml of dry dichloromethane under an inert atmosphere and an excess of oxalyl chloride (1 ml) was added. This solution was stirred at room temperature for 90 minutes and finally evaporated to dryness at elevated temperature. The brownish residue was dissolved in 50 ml of dry dichloromethane under an inert atmosphere and an excess of tin(IV) chloride (0.5 ml) was added. After stirring at room temperature for ten minutes, the reaction was quenched by slowly adding a saturated aqueous sodium bicarbonate solution. Extraction with dichloromethane furnished a green solution which was washed with water, dried over magnesium sulfate and evaporated to dryness to give Cu-1-CO (copper(II) $5^4$, $10^4$, $15^4$, $20^4$-tetra-t-butyl [3,$5^2$]-ethano-$5^6$-methyl-$3^1$-oxo-5,10,15,20-tetraphenylporphyrin).

**7.** Cu-1-CO (copper(II) $5^4$, $10^4$, $15^4$, $20^4$-tetra-t-butyl [3,$5^2$]-ethano-$5^6$-methyl-$3^1$-oxo-5,10,15,20-tetraphenylporphyrin), which was obtained by step 6, was taken up in 10 ml of trifluoroacetic acid and 2 ml of concentrated sulfuric acid. The reaction mixture was stirred at room temperature for 30 minutes. After addition of water and dichloromethane the layers were separated, the organic layer washed with water, aqueous sodium bicarbonate solution and water. After drying over magnesium sulfate the solution was evaporated to dryness to yield a green-violet solid. This was chromatographed over silica using dichloromethane and hexanes (ratio=1:2) as eluent giving FB-1-CO ($5^4$, $10^4$, $15^4$, $20^4$-tetra-t-butyl [3,$5^2$]-ethano-$5^6$-methyl-$3^1$-oxo-5,10,15,20-tetraphenylporphyrin).as a dark green powder. (yield: 107 mg, 120 μmol, 57 % after steps 1-7).

*Characterization data for FB-1-CO:*

**$^1$H NMR (400 MHz, $CDCl_3$, rt)**, δ [ppm] = 8.96 (s, 1 H), 8.89 (d, 1 H, $^3J$ = 4.9 Hz), 8.79 (m, 2H), 8.74, 8.67, 8.63 (3d, 1 H each, $^3J$ = 4.9 Hz), 8.32-7.69 (div. m's, 12H), 7.68 (d, 1 H, $^4J$ = 1.7 Hz), 7.35 (d, 1 H, $^4J$ = 1.7 Hz), 5.49 (d, 1 H, $^2J$ = 11.7 Hz), 4.11 (d, 1H, $^2J$ = 11.7 Hz), 1.59 (s, 9H), 1.58 (s, 18H), 1.50 (s, 9H), 1.17 (s, 3H), -1.62 (s, 2H).
**$^{13}$C NMR (100.5 MHz, $CDCl_3$, rt),** δ [ppm] = 192.4, 152.3, 151.2, 150.9, 150.8, 142.1, 138.6, 138.5, 138.1, 137.7, 136.1, 134.7, 134.3, 128.3, 126.1, 125.8, 124.6, 124.2, 124.0, 123.9, 123.8, 122.0, 119.0, 114.0, 53.7, 34.9, 34.8, 34.7, 31.6, 31.4, 22.5.
MS (FAB, NBA), m/z = 893.
**UV/Vis ($CH_2Cl_2$),** λ [nm] (ε [l·mor$^{-1}$·cm$^{-1}$]) = 442 (223000), 543 (11300), 587 (9000), 626 (4100), 689 (9000).
**IR (KBr),** $\tilde{\nu}$[cm$^{-1}$] = 3316, 3029, 2957, 2903, 2868, 1683, 1606, 1552, 1498, 1475, 1393, 1363, 1351, 1262, 1239, 1197, 1154, 1108, 1019, 984, 965, 869, 850, 799, 718.

| | | | | |
|---|---|---|---|---|
| **EA:** $C_{63}H_{64}N_4O \times 2H_2O$: | calculated | C 81.43 | H 7.38 | N 6.03 |
| | found | C 81.26 | H 7.54 | N 5.74 |

Example 2

Preparation of $5^4$, $10^4$, $15^4$, $20^4$-tetra-t-butyl-[3,$5^2$],[$5^6$,9]-bis-ethano-$3^1$,$9^1$-dioxo-5,10,15,20-tetraphenylporphyrin (FB-2-CO)

*Brief summary of the synthesis of FB-1-CO:*

The bis-bromomethyl porphyrin Zn-2-Br (zinc $5^2$,$5^6$-bis-(bromomethyl)-$5^4$, $10^4$, $15^4$, $20^4$-tetra-t-butyl-5,10,15,20-tetraphenylporphyrin) was reacted with potassium cyanide in PEG400 to give the dicyano porphyrin Zn-2-CN (zinc $5^4$, $10^4$, $15^4$, $20^4$-tetra-t-butyl-$5^2$,$5^6$-bis-(cyanomethyl)-5,10,15,20-tetraphenylporphyrin). Demetallation (FB-2-CN) and acidic saponification of the nitrile substituents gave the dicarboxy porphyrin FB-2-COOH ($5^4$, $10^4$, $15^4$, $20^4$-tetra-t-butyl-5,10,15,20-tetraphenylporphyrin-$5^2$,$5^6$-bis-ethanoic acid) which after metallation with cupper(II) chloride gave the copper porphyrin Cu-2-COOH (copper(II) $5^4$, $10^4$, $15^4$, $20^4$-tetra-t-butyl-5,10,15,20-tetraphenylporphyrin-$5^2$,$5^6$-bis-ethanoic acid). The acid was transformed into the acid chloride (not isolated) with oxalyl chloride and reacted with tin(IV) chloride to give the copper complex Cu-2-CO (copper(II) $5^4$, $10^4$, $15^4$, $20^4$ -tetra-t-butyl-[3,$5^2$],[$5^6$,9]-bis-ethano-$3^1$,$9^1$-dioxo-5,10,15,20-tetraphenylporphyrin). Demetallation with acid gave the desired free-base system FB-2-CO ($5^4$, $10^4$, $15^4$, $20^4$-tetra-t-butyl-[3,$5^2$],[$5^6$,9]-bis-ethano-$3^1$,$9^1$-dioxo-5,10,15,20-tetraphenylporphyrin).

*Protocol:*

The synthesis of FB-2-CO was exactly performed in the same way as the generation of FB-1-CO. Reaction conditions and yields are comparable.

*Characterization data for FB-2-CO:*

**$^1$H NMR (400 MHz, $CDCl_3$, rt)**, δ [ppm] = 8.69 (s, 2H), 8.64 (d, 2H, $^3J$ = 4.9 Hz), 8.54 (d, 2H, $^3J$ = 4.9 Hz), 8.06 (d, 6H, $^3J$ = 7.9 Hz), 7.76 (d, 6H, $^3J$ = 7.9 Hz), 7.70 (s, 2H), 4.37 (d, 2H, $^2J$ = 11.3 Hz), 3.84 (d, 2H, $^2J$ = 11.3 Hz), 1.58 (s, 18H), 1.58 (s, 9H), 1.51 (s, 9H), -1.33 (s, 2H).
**$^{13}$C NMR (100.5 MHz, $CDCl_3$, rt),** δ [ppm] = 190.0, 153.8, 151.6, 151.3, 150.8, 137.7, 137.5, 136.0, 134.7, 128.9, 127.8, 125.6, 124.3, 124.2, 122.9, 111.8, 53.9, 35.0, 34.9, 31.6, 31.3.
MS (FAB, NBA): m/z = 920.
**UV/Vis ($CH_2Cl_2$),** λ [nm] (ε [l·mor$^{-1}$·cm$^{-1}$]) = 349 (28500), 462 (103700), 486 (70200), 597 (6500), 627 (12000), 733

(11200).
**IR (KBr)**, $\tilde{\nu}$[cm$^{-1}$] = 3034, 2961, 2907, 2868, 1695, 1552, 1502, 1459, 1393, 1363, 1247, 1197, 1135, 1108, 1027, 1000, 980, 965, 869, 849, 799, 714.

| | | | | |
|---|---|---|---|---|
| **EA**: $C_{64}H_{64}N_4O_2 \times 2H_2O$: | calculated | C 80.47 | H 6.96 | N 5.87 |
| | found | C 80.28 | H 6.93 | N 5.70 |

Example 3

Determination of the photophysical properties of compound FB-1-CO prepared in Example 1.

The ground state absorption spectra were recorded at room temperature using a commercial spectrophotometer Shimadzu UV-2501 PC. Steady-state fluorescence spectra were measured in 1 cm × 1 cm quartz optical cells using a combination of a cw-Xenon lamp (XBO 150) and a monochromator (Lot-Oriel, bandwidth 10 nm) for excitation and a polychromator with a cooled CCD matrix as a detector system (Lot-Oriel, Instaspec IV).

Photosensitizer-generated time-resolved singlet oxygen luminescence was measured at 1270 nm. A nanosecond Nd-YAG laser (BMI) equipped with an OPO (BMI) was used to excite the samples at 510 nm and the luminescence signal was recorded by a germanium pin diode (Northcoast). To calculate the singlet oxygen quantum yield, $\Phi_\Delta$, the solution of Pheo in DMF as well as in ethanol were used as reference ($\Phi_\Delta^{Pheo}$ = 0.52).

To measure transient absorption spectra (TAS), a white light continuum was generated as a test beam in a cell with $D_2O/H_2O$ mixture using intense 25 ps single pulses from a Nd:YAG laser (Ekspla) at 1064 nm. Before passing through the sample, the continuum radiation was split to get a reference spectrum. The transmitted as well as the reference beam were focused into two optical fibers and were recorded simultaneously at different traces on a CCD-matrix (Lot-Oriel, Instaspec IV). The tunable radiation of a OPO/OPG (Ekspla, tuning range 210-2300 nm) pumped by the third harmonic of the same laser was used for excitation of the samples. The mechanical delay line allowed measuring the light-induced changes in the absorption spectrum at different time delays up to 15 ns after excitation.

Thus, the following photophysical properties have been determined:

| | |
|---|---|
| $Q_y$ - absorption at $\lambda$ = 690 nm fluorescence quantum yield: | $\Phi_{Fl}$ = 0,02 |
| singlet oxygen quantum yield: | $\Phi_\Delta$ = 0,9 |
| ISC quantum yield: | $\Phi_{ISC}$ = 0,95 ± 0,05 |

Example 4

Demonstration of the phototoxity of compound FB-1-CO prepared in Example 1 **Cell culture and incubation conditions**

A special line of human T-lymphocytes (Jurkat cells: clone E 6-1, human acute T-cell leukaemia, ATCC-Catalogue) were cultivated in 50 ml-flasks in 8 ml RPMI 1640 medium containing Glutamax-I, supplemented with 10% fetal calf serum (FCS), 100 g/ml streptomycin, 100 I.E./ml penicillin and 25 I.E./ml nystatin. Cells were cultivated at 37° C in 100% humidity and 5%
CO2 and were seeded in new medium every 2-3 days. Jurkat cells (2 · 105 cells/ml) were incubated in medium with 0.5% DMF solution of the studied derivative. The final extracellular concentration was of 55μM FB-1-CO. After 24h incubation time the intracellular concentration of FB-1-CO was 10μM. Cells incubated with 0.5% DMF were used as reference.
The compound FB-1-CO shows no dark cytotoxicity.

**Irradiation conditions**

For the cell irradiation experiments, a special setup was used. After incubation with or without sensitizers, cells were washed twice and placed in a 96-well culture plate (Falcon), 100 μl/well. The 96-well culture plate was positioned on a plane having a circular hole with a surface of 0.32 cm$^2$, which correspond to the surface of a single well. A laser diode with emission at the wavelength 668 nm was embedded in a cylinder whose walls were covered with barium sulphate and fixed on the bottom of the plane in correspondence to the wall. The output of the laser through the hole and a culture plate cover was of 0.60 mW, so that the irradiance resulted to be 2.12 mW/cm$^2$. Irradiation times of 0.5, 1 and 5 minutes were used. Fresh medium was added and cells were incubated at 37° C, 5% CO2 and 100% humidity

prior further analysis.

**Photo-induced cytotoxicity**

Cell membrane disruption and necrosis were detected by cell staining with the vital dye trypan blue (0.4% vol/vol) for 10 min. A very effective phototoxity has been found.

## Claims

1. A compound of formula I

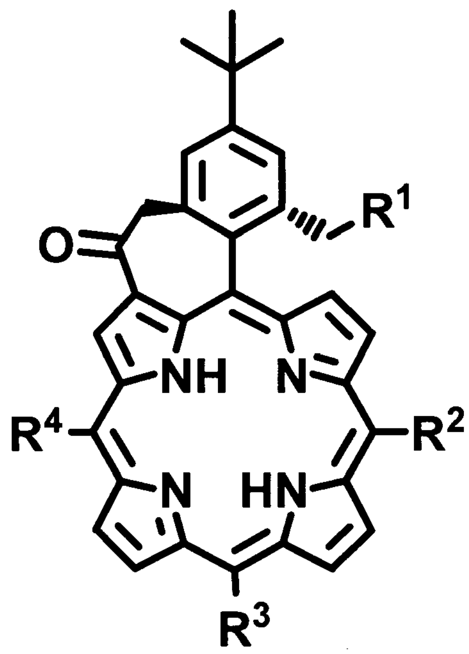

**(I)**

wherein
$R^1$ represents hydrogen, $COR^5$, $CO_2R^5$, $CONR^5R^6$, $SO_2R^5$, $SO_2NR^5R^6$ or
$R^1$ represents a carboxy group which C-atom is covalently bound to the C-atom of position 7 of the porphyrin chromophor
$R^2$, $R^3$ and $R^4$ are indepentently selected from unsubstituted or substituted $C_1$-$C_6$ alkyl, unsubstituted or substituted aryl groups
$R^5$ and $R^6$ are independently selected from hydrogen, unsubstituted or substituted $C_1$-$C_6$ alkyl, unsubstituted or substituted aryl, halogen, $C_1$-$C_5$ alkoxy, alkylether, carboxylic acid or acid salts, carboxylic acid esters, amino, nitro, amino acids or their derivates, a dendrimer moiety, a micelle forming detergent, a lipid, an antibody or antibody fragment, polypropylene glycol, polyethylene glycol (PEG), 2-hydroxypropyl methacrylate, polyvinylpyrrolidone (PVP), a pharmaceutically acceptable salt.

2. The compound according to claim 1, wherein $R^1$ represents hydrogen.

3. The compound according to claim 1, wherein $R^1$ represents a carboxy group which C-atom is covalently bound to the C-atom of position 7 of the porphyrin macrocycle.

4. The compound according to claims 1 to 3, wherein $R^2$, $R^3$ and $R^4$ are independently selected from an unsubstituted or substituted aryl ring.

5. The compound according to claim 4, wherein the aryl group is phenyl.

**6.** The compound according to claim 5, wherein phenyl is substituted with tert-butyl, preferably in 4-position.

**7.** The compound according to claims 1 to 3, wherein $R^2$, $R^3$ and $R^4$ are independently selected from $C_1$-$C_4$ alkyl.

**8.** A compound according to claims 1 to 7 for use as photosensitizer in photodynamic therapy.

**9.** Use of a compound according to claims 1 to 7 for the preparation of a pharmaceutical composition for photodynamic therapy.

**10.** Use according to claim 8 for photodynamic therapy of tumors, dermatological disorders, viral or bacterial infections, ophthalmological disorders, cardiovascular disorders or urological disorders.

**11.** A pharmaceutical composition comprising as active ingredient a compound according to claims 1 to 7.

Figure 1

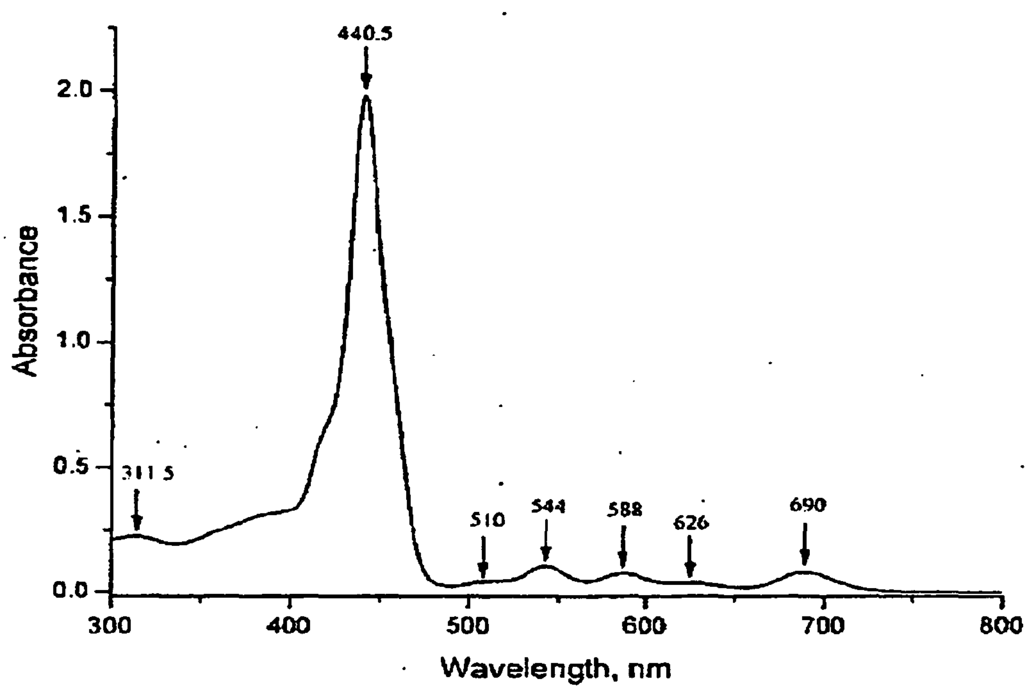
440.5
311.5
510
544
588
626
690
2.0
1.5
1.0
0.5
0.0
Absorbance
300
400
500
600
700
800
Wavelength, nm

Figure 2

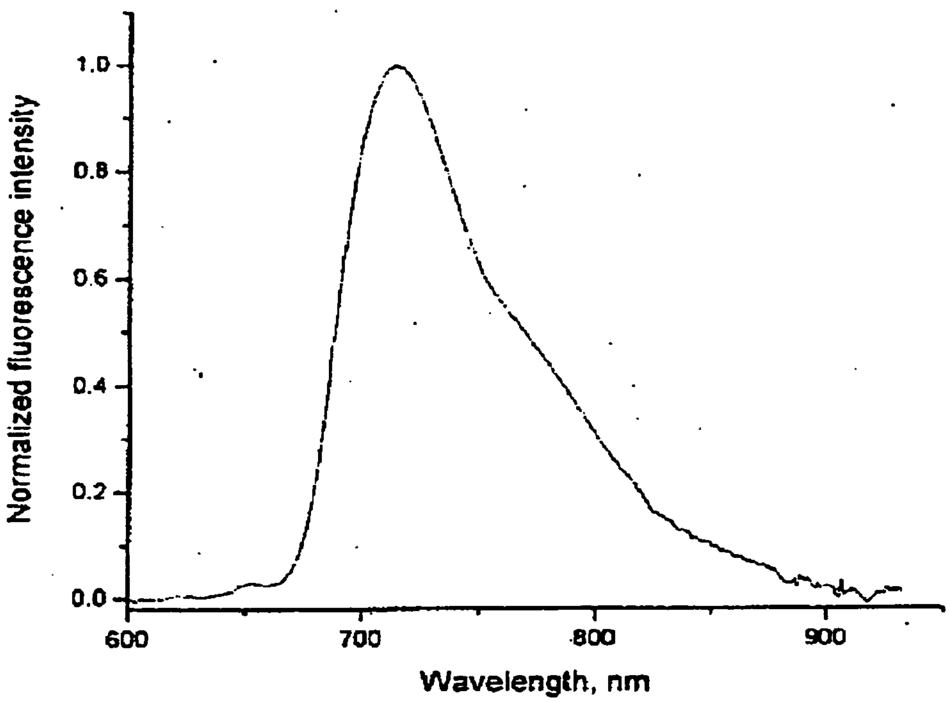
Normalized fluorescence intensity
1.0
0.8
0.6
0.4
0.2
0.0
600
700
800
900
Wavelength, nm

Figure 3

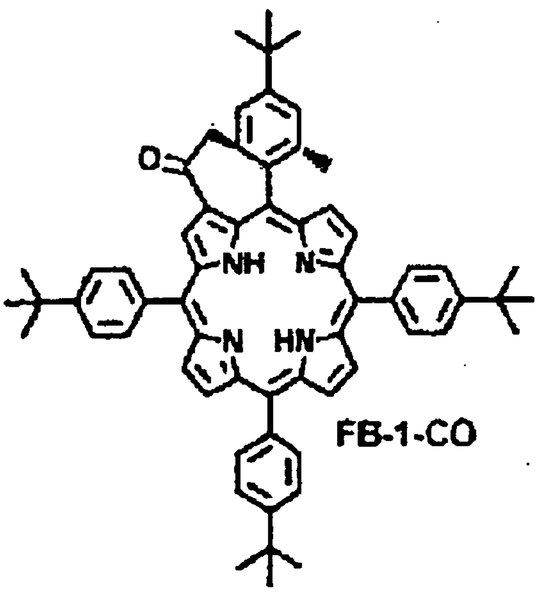

FB-1-CO

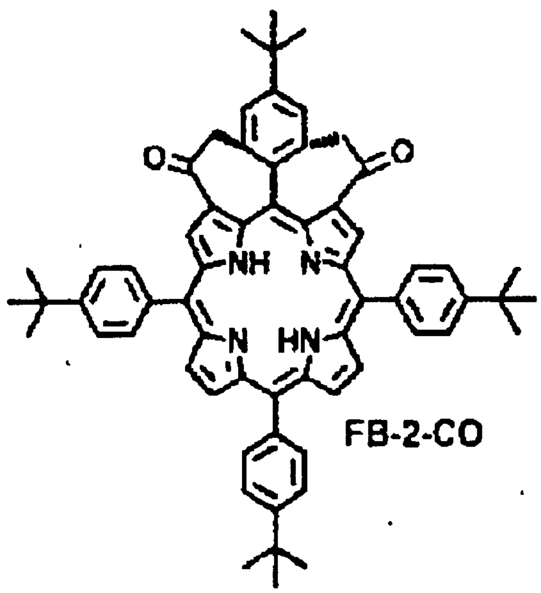

FB-2-CO

Figure 4: Synthesis of FB-1-CO

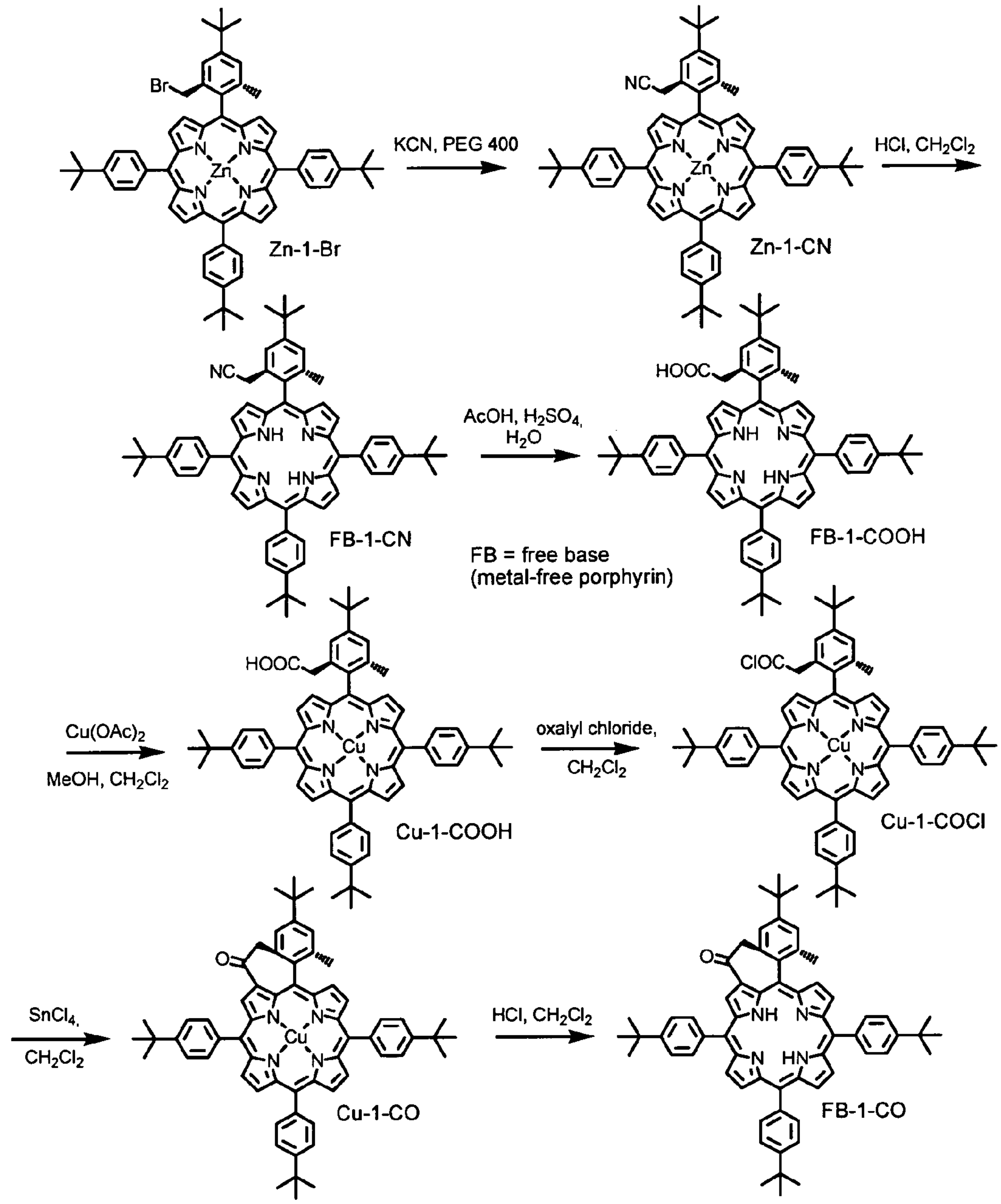


Zn-1-Br

Zn-1-CN

FB-1-CN

FB = free base (metal-free porphyrin)

FB-1-COOH

Cu-1-COOH

Cu-1-COCl

Cu-1-CO

FB-1-CO

Figure 5: Synthesis of FB-2-CO

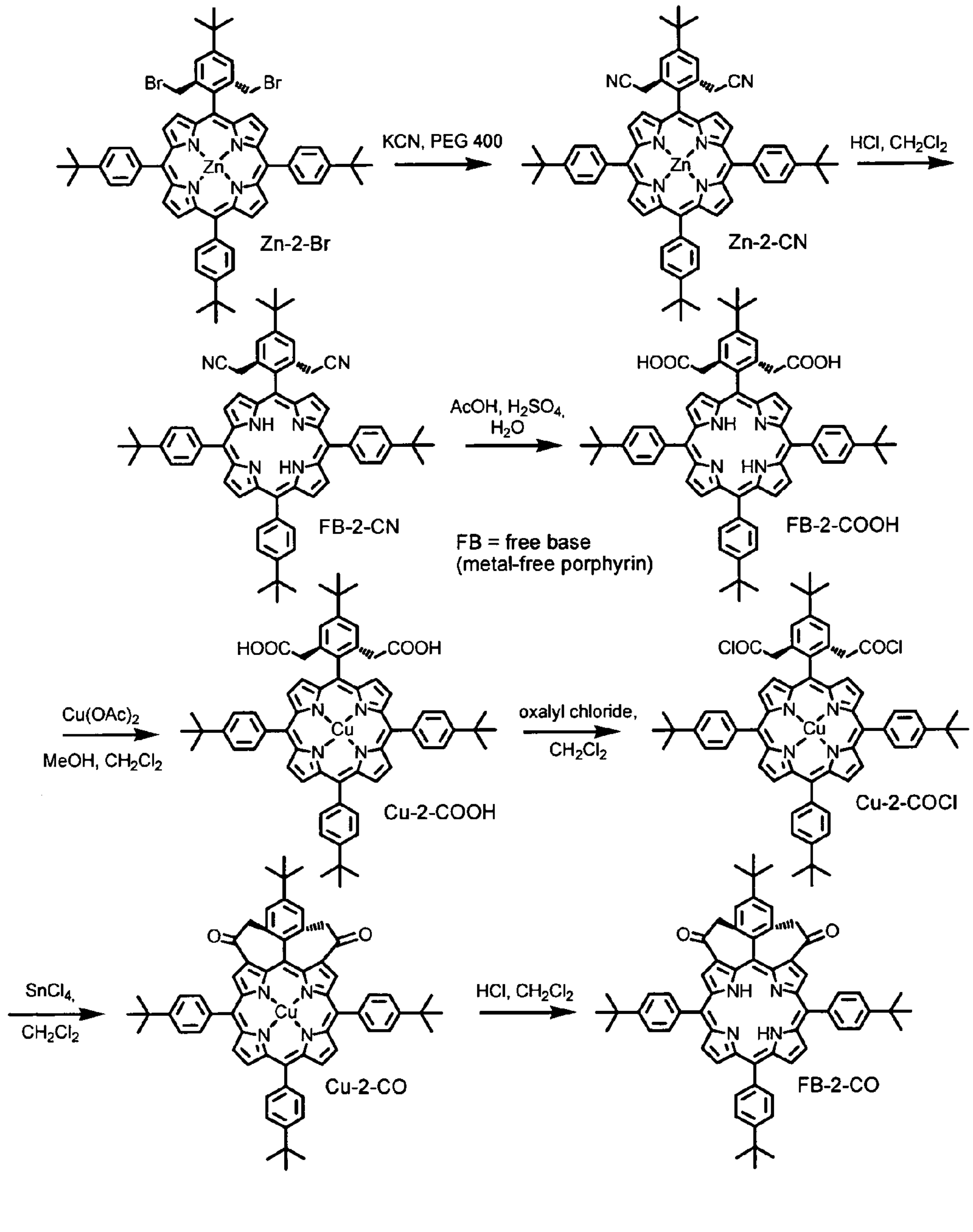

European Patent Office

# EUROPEAN SEARCH REPORT

Application Number
EP 06 07 5595

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2005/058909 A (PHOTOBIOCHEM N.V; LUGTENBURG, JOHANNIS; VAN DER HAAS, HENDRIKUS, NICOL) 30 June 2005 (2005-06-30) * claim 1 * | 1-11 | INV. C07D487/22 A61K31/40 A61P35/00 |
| A | PATENT ABSTRACTS OF JAPAN vol. 2003, no. 12, 5 December 2003 (2003-12-05) & JP 2005 104905 A (RIKOGAKU SHINKOKAI), 21 April 2005 (2005-04-21) * abstract * | 1-11 | |
| A | US 5 506 255 A (SMITH ET AL) 9 April 1996 (1996-04-09) * the whole document * | 1-11 | |
| A | LIDDELL, P.A. ET AL.: "A New Porphyrin Derivative for Use as a Diene in the Diels-Alder Reaction" TETRAHEDRON LETTERS, vol. 35, 1994, pages 995-998, XP002390097 * example 5 * | 1-11 | TECHNICAL FIELDS SEARCHED (IPC): C07D |

The present search report has been drawn up for all claims

| Place of search | Date of completion of the search | Examiner |
|---|---|---|
| Munich | 13 July 2006 | Baston, E |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 06 07 5595

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2006

| Patent document cited in search report | Publication date | Patent family member(s) | Publication date |
|---|---|---|---|
| WO 2005058909 A | 30-06-2005 | NL 1025049 C2 | 21-06-2005 |
| JP 2005104905 A | 21-04-2005 | NONE | |
| US 5506255 A | 09-04-1996 | AU 3792293 A | 13-09-1993 |
| | | WO 9316692 A1 | 02-09-1993 |
| | | US 5330741 A | 19-07-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0174818 A
- DD 248282 A1
- WO 0108704 A2


### Non-patent literature cited in the description


- **BLANT, S. A.** In vivo fluence rate effect in photodynamic therapy of early cancers with tetra(m-hydroxyphenyl)chlorin. *Photochem. Photobiol.,* 1996, vol. 64, 963-968
- **RICHETER, S.** *J. Org. Chem.,* 2003, vol. 68, 9200-9208
- Photodynamic Therapy. Encyclopedia of Analytical Chemistry. John Wiley & Sons Ltd, 2000, 302-322
- **N. JUX.** o-(Bromomethyl)Substituted Tetraarylporphyrin Building Blocks. *Org. Lett.,* 2000, vol. 2, 2129-2132
- **NGA HOANG HUYEN ; ULRIKE JANNSEN ; HANAA MANSOUR ; NORBERT JUX.** Introducing the Staudinger phosphazene reaction to porphyrin chemistry. *J. Porphyrins Phthalocyanines,* 2004
- **D. DOLPHIN.** The Porphyrins. Academic Press, 1978, vol. 1, 88-90
- **GUNTER, M. J. ; MANDER, L. N.** *J. Org. Chem.,* 1981, vol. 46, 4792-4795
- Synthesis of meso-Substituted Porphyrins. **J. S. LINDSEY.** The Porphyrin Handbook. Academic Press, 2000, vol. 1, 45-112
- **S. HACKBARTH.** *Optics Communications,* 2005, vol. 248, 295-306
- **NORBERT JUX.** o-(Bromomethyl)Substituted Tetraaryl-porphyrin Building Blocks. *Org. Lett.,* 2000, vol. 2, 2129-2132
- **NGA HOANG HUYEN ; ULRIKE JANNSEN ; HANAA MANSOUR ; NORBERT JUX.** Introducing the Staudinger phosphazene reaction to porphyrin chemistry. *J. Porphyrins Phthalocyanines,* 2004, vol. 8, 1356-1365